# EUROPEAN PATENT APPLICATION

(11) **EP 4 273 256 A1**
(43) Date of publication of application: **08.11.2023**
(21) Application number: 21924452.2
(22) Date of filing: 24.12.2021
(51) Int. Cl.: C12P 17/16, C12R 1/39, C12N 1/20

(54) **FERMENTATION METHOD FOR MUPIROCIN**

(30) Priority: 07.02.2021 CN 202110168751; 30.03.2021 CN 202110337621; 18.06.2021 CN 202110678427
(71) Applicant: Hangzhou Zhongmeihuadong Pharmaceutical Co., Ltd., GongShu District HangZhou, Zhejiang 310011 (CN)
(72) Inventor: CHENG, Zhaobing, Hangzhou, Zhejiang 310011 (CN); KOU, Guanghui, Hangzhou, Zhejiang 310011 (CN); XU, Yiming, Hangzhou, Zhejiang 310011 (CN); HU, Weijie, Hangzhou, Zhejiang 310011 (CN); ZHANG, Yuchen, Hangzhou, Zhejiang 310011 (CN)
(74) Representative: HGF
(86) International application number: PCT/CN2021/141025
(87) International publication number: WO 2022/166459

(57) **Abstract**

A fermentation method for mupirocin, the method comprising performing shake flask seed culture, seed tank culture, fermentation culture, feed culture. The fermentation method is not only suitable for industrial mass production, but also improves the fermentation potency of mupirocin, which can be stably maintained to be 8000 ug/ml or above.

## Description

### TECHNICAL FIELD

The present invention relates to the technical field of pharmaceuticals, particularly to the technical field of fermentation, and more particularly to a fermentation method for mupirocin comprising seed culture, fermentation culture, feed culture, etc.

### BACKGROUND

Mupirocin (pseudomonic acid A), is an aminoacyl transaminase inhibitor antibiotic that specifically binds to the isoleucyl-tRNA synthetase in bacteria, thereby inhibiting the synthesis of isoleucine-containing proteins in bacteria. It can effectively resist various gram-positive bacteria including *Staphylococcus aureus*, *Streptococcus fermentans*, *Staphylococcus epidermidis*, *Streptococcus pneumoniae*, *Streptococcus pyogenes*, etc., and some gram-negative bacteria including *Haemophilus influenzae*, etc. Mupirocin has no cross-drug resistance with other antibiotics, demonstrating higher medical value. It has long been clinically applied, and is sold under the trade name Bactroban^{®} or mupirocin ointment.

Patent No. WO2000046389 discloses a method for preparing pseudomonic acid A, comprising culturing a pseudomonas strain capable of biosynthesizing pseudomonic acid A on a medium comprising an organic nitrogen source or a carbon source and completing the culture, which involves a medium of complex components and an unstable potency. Patent No. CN201410083050.0 discloses a method for preparing pseudomonic acid A and conditions for a series of cultures and fermentations. The procedures of the method are complicated and the potency is unstable. It is uncertain whether the method can be put into practical application. Patent No. CN201810896368.9 discloses a fermentation medium of mupirocin which additionally contains a growth promoter. However, the potency is not good and it is not suitable for mass production.

Existing fermentation methods for mupirocin have unsatisfactory aspects in terms of strict process control conditions, environmental requirements and commercial or ecological perspectives. Therefore, there is still a need for new fermentation methods for mupirocin that are suitable for industrial mass production and are environment-friendly, cost-efficient.

### SUMMARY

Mupirocin, or pseudomonic acid A, is produced by the fermentation of *Pseudomonas fluorescens.* Existing fermentation methods for mupirocin are unstable and low in potency, and thus are not suitable for industrial mass production. The present invention provides a fermentation method for mupirocin suitable for industrial mass production, comprising shake flask seed culture, seed tank culture, fermentation culture and feed culture.

The fermentation method for mupirocin of the present invention is suitable for mass production and is environment-friendly. Mupirocin produced by the fermentation method of the present invention may have a stable potency of more than 8000 µg/mL.

The fermentation medium of mupirocin comprises the following components:

| Fermentation medium components | Mass-to-volume ratio (W/V) |
|---|---|
| Carbon source | 5∼12% |
| Nitrogen source | 2∼10% |
| Inorganic salt | 0.1∼1.5% |
| Defoamer | 0.1∼0.5% |

The pH of the fermentation medium is 6.0-7.5.

The fermentation medium is prepared according to the above formula. Water is added into a fermenter, and the materials are added into the fermenter while stirring.

The volume of the fermentation feed may be 0-20000 L.

The time of the fermentation culture is 48-144 h, preferably 80-132 h.

The temperature of the fermentation tank is 10-37 °C, preferably 20-30 °C.

Feeding is started after 24 h of the fermentation culture. After feeding, if the pH increases, the feeding amount of sugar is properly increased, and if the pH is stable, the feeding amount of sugar is maintained or properly reduced.

The formula of the feed is as follows:

| Feed components | Mass-to-volume ratio (W/V) |
|---|---|
| Carbon source | 25∼60% |
| Nitrogen source | 1∼10% |
| Inorganic salt | 0.10∼0.5% |
| Defoamer | 0.1∼0.5% |

The pH after feeding is 5.0-6.5.

As a specific embodiment, the carbon source includes but is not limited to: glucose, sucrose, fructose, glycerol, dextrin, peptone, wheat bran, bean cake powder, corn starch, soybean powder, potato powder, tapioca starch, corn steep liquor, yeast powder, mannitol, and the like.

As a specific embodiment, the nitrogen source includes but is not limited to: ammonia solution, urea, ammonium salt, nitrate, peptone, wheat bran, corn steep liquor, bean cake powder, peanut cake powder, cottonseed cake powder, yeast powder, and the like.

As a specific embodiment, the inorganic salt includes but is not limited to: sulfate, phosphate, chloride, and the like.

The present invention also provides a fermentation method for mupirocin, comprising:
1. Shake flask seed culture: comprising preparing a shake flask seed, split charging sterilizing, inoculating and incubating to obtain a shake flask seed bacterial solution;
2. Seed tank culture: comprising preparing a medium for the seed, sterilizing, inoculating and incubating to obtain a seed bacterial solution;
3. Fermentation culture: comprising preparing a fermentation medium, sterilizing, inoculating and incubating to obtain a mupirocin fermentation broth;
4. Feed culture: comprising preparing a feed medium, sterilizing and incubating to obtain a mupirocin fermentation broth;
5. Harvest.

The medium formula of the shake flask seed culture is as follows:

| Shake flask seed culture medium components | Mass-to-volume ratio (W/V) |
|---|---|
| Carbon source | 1∼5% |
| Nitrogen source | 0.5∼1.5% |
| Inorganic salt | 0.1∼0.5% |
| Defoamer | 0.02∼0.1% |

The pH of the shake flask seed culture is 6.0-7.5.

The temperature of the shake flask seed culture is 20-30 °C.

The culture time of the shake flask seed is 18-30 h, preferably 20-24 h.

The shake flask seed is inoculated into a seed tank at an inoculation amount of 0. 1-10%.

The culture medium formula of the seed tank culture is as follows:

| Seed tank culture medium components | Mass-to-volume ratio (W/V) |
|---|---|
| Carbon source | 1∼5% |
| Nitrogen source | 0.5∼1.5% |
| Inorganic salt | 0.1∼0.5% |
| Defoamer | 0.01∼0.1% |

The pH of the seed tank culture is 6.0-7.5.

The temperature of the seed tank culture is 20-30 °C.

The culture time of the seed tank culture is 12-36 h, preferably 18-24 h.

The carbon source in the culture medium includes but is not limited to: glucose, sucrose, fructose, glycerol, dextrin, peptone, wheat bran, bean cake powder, corn starch, soybean powder, potato powder, tapioca starch, corn steep liquor, yeast powder, mannitol, and the like.

The nitrogen source in the culture medium includes but is not limited to: ammonia solution, urea, ammonium salt, nitrate, peptone, wheat bran, corn steep liquor, bean cake powder, peanut cake powder, cottonseed cake powder, yeast powder, and the like.

The inorganic salt in the culture medium includes but is not limited to: sulfate, phosphate, chloride, and the like.

As a specific embodiment, the formula of the fermentation medium of the present invention is as follows:

| Fermentation medium components | Mass-to-volume ratio (W/V) |
|---|---|
| Glucose | 4.0∼10.0%; |
| Bean cake powder | 1.5∼3.0%; |
| Wheat bran | 0.5∼2.5%; |
| Urea | 0.1∼0.2%; |
| Glycerol | 1.0∼2.0%; |
| Ammonium sulfate | 0.01∼1.0%; |
| Potassium dihydrogen phosphate | 0.01∼0.25%; |
| Magnesium sulfate | 0.01∼0.25%; |
| Potassium chloride | 0.01∼0.25%; |
| Zinc sulfate | 0.01∼0.25%; |
| Polyether GPE defoamer | 0.1∼0.5%. |

As a specific embodiment, the fermentation culture comprises: mixing each component according to the ratio, adding water into a fermenter, adding the materials into the fermenter while stirring, adjusting pH to 6.8-7.5 with sodium hydroxide, sterilizing the tank and pipes in sequence, and pressing the seed bacterial solution into the fermenter with sterile air.

As a specific embodiment, the formula of the feed of the present invention is as follows:

| Feed components | Mass-to-volume ratio (W/V) |
|---|---|
| Glucose | 25∼60%; |
| Bean cake powder | 1∼5%; |
| Wheat bran | 1∼5%; |
| Glycerol | 0.1∼1.0%; |
| Potassium dihydrogen phosphate | 0.01∼0.1%; |
| Magnesium sulfate | 0.01∼0.1%; |
| Zinc sulfate | 0.01∼0.1%; |
| Polyether GPE defoamer | 0.1∼0.5%. |

As a specific embodiment, the feed culture comprises: mixing each component according to the ratio, transferring the seed after sterilizing the tank and pipes in sequence, and monitoring the pH and the content of glucose regularly after transferring the seed.

As a specific embodiment, the formula of the shake flask seed of the present invention is as follows:

| Shake flask seed components | Mass-to-volume ratio (W/V) |
|---|---|
| Glucose | 0.5∼5%; |
| Wheat bran | 0.2∼1.0%; |
| Glycerol | 0.2∼1.0%; |
| Ammonium sulfate | 0.1∼0.5%; |
| Magnesium sulfate | 0.01∼0.1%; |
| Potassium dihydrogen phosphate | 0.01∼0.1%; |
| Polyether GPE defoamer | 0.01∼0.1%. |

As a specific embodiment, the shake flask seed culture comprises: mixing each component according to the ratio, adding an appropriate amount of water, stirring, adjusting pH to 6.8-7.5 with sodium hydroxide solution, taking a slant medium after sterilization, washing with sterile water, inoculating into a seed shake flask at an inoculation amount of 0. 1-10.0%, wherein the culture temperature of the seed shake flask is 20-30 °C, the culture time is 12-48 h.

As a specific embodiment, the formula of the seed tank culture medium of the present invention is as follows:

| Seed tank culture medium components | Mass-to-volume ratio (W/V) |
|---|---|
| Glucose | 0.5∼5%; |
| Wheat bran | 0.2∼1.0%; |
| Glycerol | 0.2∼1.0%; |
| Ammonium sulfate | 0.1∼0.5%; |
| Magnesium sulfate | 0.01∼0.1%; |
| Potassium dihydrogen phosphate | 0.01∼0.1%; |
| Polyether GPE defoamer | 0.1∼0.5%. |

As a specific embodiment, the seed tank culture comprises: mixing each component according to the ratio, adding water into a seed tank, adding the materials while stirring, adjusting pH to 6.8-7.5 with sodium hydroxide, inoculating the shake flask seed bacterial solution into the seed tank after sterilization, wherein the temperature of the seed tank is 20-30 °C during inoculation. The culture time is 12-48 h, the pH of the culture is 6.8-7.5.

As a specific embodiment, a harvest is started after 80-132 h of the fermentation culture and/or feed culture, wherein the harvest potency is greater than or equal to 8000 ng/mL. The monitoring and control of the pH and glucose are stopped before the harvest. The fermentation broth is transferred into a pretreatment tank for subsequent extraction and/or purification.

As a specific embodiment, when the fermentation is finished, the pH of harvest is greater than 6.0, the content of glucose is 0.5-6% W/V.

As a specific embodiment, when the shake flask seed is inoculated into the seed tank, the content of glucose is 0.1-5% W/V, preferably 0.5-1.5% W/V. when the shake flask seed is inoculated into the seed tank, the pH is 6.0-7.0.

As a specific embodiment, when the seed bacterial solution is pressed into the fermenter, the inoculation amount is 1.0-8.0%, preferably 2.5-5%.

As a specific embodiment, before the seed bacterial solution is pressed into the fermenter, the pH is 6.0-6.5; the content of glucose in the fermentation medium is 3.0-7.0% W/V, preferably 4.0-6.5% W/V

As a specific embodiment, the shake flask seed culture comprises: weighing, in mass-to-volume ratio, 1.0% of glucose, 0.5% of wheat bran, 0.5% of glycerol, 0.05% of magnesium sulfate, 0.05% of potassium dihydrogen phosphate, 0.2% of ammonium sulfate, 0.05% of polyether GPE defoamer and the remaining of drinking water, stirring, adjusting pH to 6.8-7.5 with sodium hydroxide solution, preparing a slant medium after sterilization, washing with sterile water, inoculating into a seed shake flask at an inoculation amount of 0.1-10.0%, wherein the temperature of the seed shake flask culture is 20-30 °C, the system is incubated with shaking for 12-48 h.

As a specific embodiment, the seed tank culture comprises: weighing, in mass-to-volume ratio, 1.0% of glucose, 0.5% of wheat bran, 0.5% of glycerol, 0.05% of magnesium sulfate, 0.05% of potassium dihydrogen phosphate, 0.2% of ammonium sulfate and 0.05% of polyether GPE defoamer; adding water into a seed tank, adding the materials while stirring, adjusting pH to 6.8-7.5 with sodium hydroxide, and sterilizing at a tank temperature at 90-150 °C, wherein the content of glucose in the medium in the seed tank is measured 0.5-1.5% and the pH of the medium is measured 6.0-7.0 before inoculation; and inoculating the shake flask seed solution into the seed tank, wherein the temperature of the seed tank is 20-30 °C, the culture time is 12-36 h, and the pH is controlled at 6.5-7.5; under a microscope, the cells are rod-shaped, deep-stained and in a large amount with no contaminating bacteria observed.

As a specific embodiment, the fermentation culture comprises: weighing, in mass-to-volume ratio, 5.0-6.0% of glucose, 2.0% of bean cake powder, 1.5% of wheat bran, 1.5% of glycerol, 0.15% of urea, 0.5% of ammonium sulfate, 0.1% of potassium dihydrogen phosphate, 0.06% of magnesium sulfate, 0.12% of potassium chloride, 0.2% of zinc sulfate and 0.3% of polyether GPE defoamer; adding water into a fermenter, adding the materials while stirring, adjusting pH to 6.8-7.5 with sodium hydroxide, sterilizing at a fermenter temperature of 90-150 °C and keeping the temperature and the pressure of the fermenter for 0.5-1 h, wherein the content of glucose in the medium in the fermenter is measured 4.0-6.5% and the pH of the medium is measured 6.0-7.0 before inoculation; pressing the seed bacterial solution into the fermenter with sterile air at an inoculation amount of 1.0-8.0%, wherein the temperature of the fermenter is 20-30 °C; the pH and the content of glucose in the fermentation broth during the fermentation and metabolism process are the main control parameters.

As a specific embodiment, the feed culture comprises: taking samples regularly after the beginning of the fermentation culture for assay of glucose content and pH, and starting the feeding when the content of glucose is lower than 1.0%, wherein the feed comprises 30-50% of glucose, 1-5% of wheat bran, 1-5% of bean cake powder, 0.5% of glycerol, 0.06% of magnesium sulfate, 0.05% of potassium dihydrogen phosphate, 0.05% of zinc sulfate and 0.3% of polyether GPE defoamer; sterilizing at a fermenter temperature of 90-150 °C; monitoring and controlling the content of glucose and pH in the fermenter repeatedly, wherein feeding is started when the content of glucose is lower than 1.0%, and after feeding, the pH is controlled at 5.0-6.0 and the content of glucose is controlled at 1.0-1.5%.

As a specific embodiment, the harvest comprises: after 80-128 h of fermentation culture, when the harvest potency is greater than or equal to 8000 µg/mL, stopping the monitoring and control of pH and glucose before the harvest. when the pH is greater than 6.0 and the content of glucose is lower than 0.5%, fermentation ends. The transferring the fermentation broth into a pretreatment tank for subsequent extraction and purification of the fermentation broth.

The fermentation method for mupirocin of the present invention comprises:
1. Shake flask seed culture: weighing, in mass-to-volume ratio, 1.0% of glucose, 0.5% of wheat bran, 0.5% of glycerol, 0.05% of magnesium sulfate, 0.05% of potassium dihydrogen phosphate, 0.2% of ammonium sulfate, 0.05% of polyether GPE defoamer and the remaining of drinking water, stirring, adjusting pH to 6.8-7.5 with sodium hydroxide solution, sterilizing at 90-150 °C for 0.5-1 h, taking a slant medium after sterilization, washing with sterile water, inoculating into a seed shake flask at an inoculation amount of 0.1-10.0%, wherein the temperature of the seed shake flask culture is 20-30 °C, and the system is incubated with shaking for 12-48 h;
2. Seed tank culture: weighing, in mass-to-volume ratio, 1.0% of glucose, 0.5% of wheat bran, 0.5% of glycerol, 0.05% of magnesium sulfate, 0.05% of potassium dihydrogen phosphate, 0.2% of ammonium sulfate and 0.05% of polyether GPE defoamer; adding water into a seed tank, adding the materials while stirring, adjusting pH to 6.8-7.5 with sodium hydroxide, and sterilizing at a tank temperature at 90-150 °C for 0.5-1 h at constant temperature and pressure, wherein the content of glucose in the medium in the seed tank is measured 0.5-1.5% and the pH of the medium is measured 6.0-7.0 before inoculation; and inoculating the shake flask seed solution into the seed tank, wherein the temperature of the seed tank is 20-30 °C, the culture time is 12-36 h, and the pH is controlled at 6.5-7.5; under a microscope, the cells are rod-shaped, deep-stained and in a large amount with no contaminating bacteria observed;
3. Fermentation culture: weighing, in mass-to-volume ratio, 5.0-6.0% of glucose, 2.0% of bean cake powder, 1.5% of wheat bran, 1.5% of glycerol, 0.15% of urea, 0.5% of ammonium sulfate, 0.1% of potassium dihydrogen phosphate, 0.06% of magnesium sulfate, 0.12% of potassium chloride, 0.2% of zinc sulfate and 0.3% of polyether GPE defoamer; adding water into a fermenter, adding the materials while stirring, adjusting pH to 6.8-7.5 with sodium hydroxide, sterilizing at a fermenter temperature of 90-150 °C and keeping the temperature and the pressure of the fermenter for 0.5-1 h, wherein the content of glucose in the medium in the fermenter is measured 4.0-6.5% and the pH of the medium is measured 6.0-7.0 before inoculation; pressing the seed solution into the fermenter with sterile air at an inoculation amount of 1.0-8.0%, wherein the temperature of the fermenter is 20-30 °C; the pH and the content of glucose in the fermentation broth during the fermentation and metabolism process are the main control parameters;
4. Feed culture: taking samples regularly after the beginning of the fermentation culture for assay of glucose content and pH, and starting the feeding when the content of glucose is lower than 1.5%, wherein the feed comprises 30-50% of glucose, 1-5% of wheat bran, 1-5% of bean cake powder, 0.5% of glycerol, 0.06% of magnesium sulfate, 0.05% of potassium dihydrogen phosphate, 0.05% of zinc sulfate and 0.3% of polyether GPE defoamer; sterilizing at a fermenter temperature of 90-150 °C at constant temperature and pressure for 0.5-1 h, and cooling to 25-40 °C after sterilization; monitoring and controlling the content of glucose and pH in the fermenter repeatedly, wherein feeding is started when the content of glucose is lower than 1.0%, and after feeding, the pH is controlled at 5.0-6.0 and the content of glucose is controlled at 1.0-1.5%.;
5. Harvest: after 80-132 h of fermentation culture, when the harvest potency is greater than or equal to 8000 µg/mL, stopping the monitoring and control of pH and glucose before the harvest; when the pH is greater than 6.0 and the content of glucose is lower than 0.5%, fermentation ends. The transferring the fermentation broth into a pretreatment tank for subsequent extraction and purification of the fermentation broth.

In the prior art of the mupirocin fermentation process, there is a method of fed-batch fermentation, wherein glucose is continuously fed into the fermentation system during the fermentation culture to supply carbon sources for the division, passage, growth and metabolism of the bacteria. In the process of microbial culture, the mupirocin fermentation process of the present invention supplements a mixed feed based on the fermentation medium, comprises a quick-acting carbon source glucose, a comprehensive carbon source glycerol, comprehensive carbon and nitrogen sources bean cake powder and wheat bran, inorganic salts potassium dihydrogen phosphate, magnesium sulfate and zinc sulfate, and a defoamer polyether GPE defoamer. That is, in fed-batch fermentation, the sole carbon source feed is replaced by a mixed feed based on the formula of the medium, and the fermentation time is properly prolonged, such that the fermentation potency is improved.

The present invention has the following beneficial effects:
1. The fermentation method for mupirocin of the present invention is suitable for small-scale experiments and kiloliter-scale industrial production. The average fermentation potency of the process may be greater than 8000 µg/mL, and the quality meets the criteria of USP 41 and EP 9.0.
2. Compared with the prior art, the present invention improves the passage and metabolism capacity of the bacteria in the fermentation process, prolongs the time to produce the potency and improves the final output of the fermentation by improving the formula of the feed in the process of fermentation culture, replacing the basic method for feeding sugar with the mixed feed adjusted based on the fermentation medium and correspondingly adjusting the fermentation period.

### BRIEF DESCRIPTION OF THE DRAWING

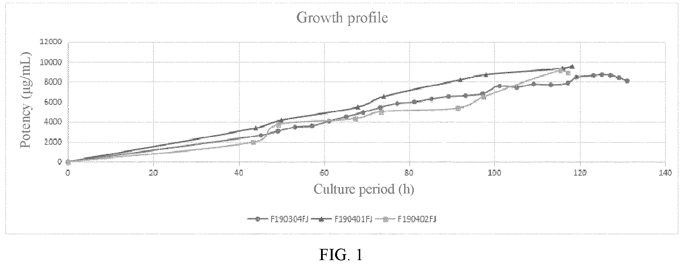

FIG. 1 illustrates the growth profile of the bacteria in the scale-up fermentation study in Example 7.

### DETAILED DESCRIPTION

The present invention will be further illustrated in detail with reference to the following specific examples. The illustration of the following examples is only intended to help understand the method of the present invention and its core concepts. Any possible changes and substitutions can be made by those skilled in the art without departing from the spirit of the present invention, and these changes and substitutions are all within the scope of the present invention. Experimental procedures without specific conditions indicated in the following examples of the present invention are generally conducted according to conventional conditions, or according to conditions recommended by the manufacturers of the starting materials or commercial products. Materials and reagents without specific sources indicated are generally commercially available conventional reagents.

### Method for preserving the strain

The strain was preserved in glycerol or on a slant medium.

### Example 1. Preparation of shake flask seed (strain culture)

Formula (W/V): 1.0% of glucose; 0.5% of wheat bran; 0.05% of magnesium sulfate; 0.05% of potassium dihydrogen phosphate; 0.2% of ammonium sulfate; 0.05% of polyether GPE defoamer; 0.5% of glycerol.

Preparation, split charging and sterilization: The materials were weighed according to the formula. 400 mL of drinking water was added. The mixture was mixed well by stirring and adjusted to pH 7.0 with sodium hydroxide solution. The volume was made up to 500 mL before the mixture was transferred into a 3000 mL conical flask and sterilized at 121 °C for 30 min.

Inoculation and culture: A slant medium was taken and washed with sterile water. The strain was inoculated into a seed shake flask at an inoculation amount of 0.1-10.0%. The temperature of the seed shake flask culture was 20-30 °C and the mixture was incubated with shaking for 24 h.

In a microscopic examination of the shake flask seed, the bacteria were rod-shaped, deep- stained and in a large amount with no contaminating bacteria observed.

### Example 2. Preparation of seed tank (seed culture)

The formula of the seed tank medium (W/V): 1.0% of glucose; 0.5% of wheat bran; 0.05% of magnesium sulfate; 0.05% of potassium dihydrogen phosphate; 0.2% of ammonium sulfate; 0.05% of polyether GPE defoamer; 0.5% of glycerol.

The materials were prepared according to the above formula. Water was added into the seed tank. The materials were added into the seed tank while stirring, and the pH was adjusted to 7.0 with 6 N sodium hydroxide solution.

The tank was sterilized with steam. The temperature of the tank was kept at 120 °C and the pressure of the tank was kept at 0.09-0.12 MPa for 30 min.

The shake flask seed bacterial solution prepared in the previous step was inoculated into the seed tank. Before inoculation, glucose in the sterilized seed tank medium should be 0.5-1.5%, and the pH should be 6.0-7.0. The pressure of the seed tank was 0.04-0.05 MPa, the temperature of the seed tank was 20-30 °C, and the pH of the seed bacterial solution was controlled at 6.5-7.5. Under a microscope, the cells were rod-shaped, deep-stained and in a large amount with no contaminating bacteria observed.

### Example 3. Preparation of fermenter (fermentation culture)

The formula of the fermentation medium (W/V): 5.0-6.0% of glucose; 2.0% of bean cake powder; 1.5% of wheat bran; 0.15% of urea; 0.5% of ammonium sulfate; 0.10% of potassium dihydrogen phosphate; 0.06% of magnesium sulfate; 0.12% of potassium chloride; 0.2% of zinc sulfate; 1.5% of glycerol; 0.3% of polyether GPE defoamer.

The materials were prepared according to the above formula. Water was added into the fermenter. The materials were added while stirring, and the pH was adjusted to 6.5-7.0 with solid sodium hydroxide.

The tank was sterilized with steam. The temperature of the tank was kept at 120 °C and the pressure of the tank was kept at 0.09-0.12 MPa for 30 min. The pipes were sterilized for 60 min. The seed bacterial solution was pressed into the fermenter with sterile air at an inoculation amount of 1.0-8.0%. The state of the seed was checked and recorded before inoculation. Before inoculation, glucose in the medium of the sterilized fermenter should be 4.0-6.5% and the pH should be 6.0-6.5. The pressure of the fermenter was 0.04-0.05 MPa and the temperature of the fermenter was 20-30 °C. The pH and the content of glucose in the fermentation broth during the fermentation and metabolism process were the main control parameters.

### Example 4. Preparation of feed (feed culture)

The formula of the feed was: 30-50% of glucose; 2.0% of wheat bran; 2.0% of bean cake powder; 0.5% of glycerol; 0.06% of magnesium sulfate; 0.05% of potassium dihydrogen phosphate; 0.05% of zinc sulfate; 0.3% of polyether GPE defoamer.

The tank was sterilized with steam. The temperature of the tank was kept at 120 °C and the pressure of the tank was kept at 0.09-0.12 MPa for 30 min. After sterilization, the temperature of the tank was reduced to and kept at 30-40 °C. The sugar-feeding pipe was sterilized for 60 min.

Samples were taken regularly after the beginning of the fermentation culture for assay of glucose and pH. Sugar feeding was started when glucose was lower than 1.0%, wherein the pH was controlled at 5.0-6.0 after sugar was fed, and glucose was controlled to be below 1.5%. Samples were taken continuously and regularly for assay of glucose and pH.

### Example 5. Investigation of carbon source and nitrogen source in fermenter and feed

### Study design:

The experiment was intended to screen the selections of carbon sources and nitrogen sources in the formula of fermentation culture and feed culture.

The investigated carbon sources, nitrogen sources and comprehensive carbon and nitrogen sources were: dextrin, peptone, wheat bran, bean cake powder, raw bean powder, corn steep liquor, yeast powder, urea and the like.

Each of the following protocols is identical to those of examples 1-4, except for the components and proportions of the carbon source and nitrogen source in the fermenter and the formula of the feed. Formulas:

### Experiment 1

The formula of the fermentation medium (W/V): 5.0-6.0% of glucose, 2.0% of yeast powder, 1.5% of wheat bran, 0.15% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of yeast powder, 1.5% of wheat bran and 0.3% of polyether GPE defoamer.

### Experiment 2

The formula of the fermentation medium (W/V): 5.0-6.0% of glucose, 2.0% of bean cake powder, 1.5% of wheat bran, 0.15% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of bean cake powder, 1.5% of wheat bran and 0.3% of polyether GPE defoamer.

### Experiment 3

The formula of the fermentation medium (W/V): 5.0-6.0% of glucose, 2.0% of peptone, 1.5% of wheat bran, 0.15% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of peptone, 1.5% of wheat bran and 0.3% of polyether GPE defoamer.

### Experiment 4

The formula of the fermentation medium (W/V): 5.0-6.0% of glucose, 2.0% of raw bean powder, 1.5% of wheat bran, 0.15% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of raw bean powder, 1.5% of wheat bran and 0.3% of polyether GPE defoamer.

### Experiment 5

The formula of the fermentation medium (W/V): 5.0-6.0% of glucose, 2.0% of bean cake powder, 1.5% of dextrin, 0.15% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of bean cake powder, 1.5% of dextrin and 0.3% of polyether GPE defoamer.

### Experiment 6

The formula of the fermentation medium (W/V): 5.0-6.0% of glucose, 2.0% of bean cake powder, 1.5% of corn steep liquor, 0.15% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of bean cake powder, 1.5% of corn steep liquor and 0.3% of polyether GPE defoamer.

### Experiment 7

The formula of the fermentation medium (W/V): 5.0-6.0% of glucose, 2.0% of bean cake powder, 1.0% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of bean cake powder, 0.5% of urea and 0.3% of polyether GPE defoamer.

### Experiment 8

The formula of the fermentation medium (W/V): 5.0-6.0% of glucose, 2.0% of raw bean powder, 1.5% of dextrin, 0.15% of urea, 0.5% of ammonium sulfate, 0.10% of potassium dihydrogen phosphate, 0.06% of magnesium sulfate, 0.12% of potassium chloride, 0.2% of zinc sulfate, 1.5% of glycerol and 0.3% of polyether GPE defoamer;

The formula of the feed (W/V): 30-50% of glucose, 0.05% of zinc sulfate, 0.06% of magnesium sulfate, 0.05% of potassium dihydrogen phosphate, 0.5% of glycerol, 1.5% of raw bean powder, 1.5% of dextrin and 0.3% of polyether GPE defoamer.

### Results:

| Formulas | Harvest potency (µg/mL) |
|---|---|
| Experiment 1 | 7977 |
| Experiment 2 | 8870 |
| Experiment 3 | 5982 |
| Experiment 4 | 8020 |
| Experiment 5 | 8535 |
| Experiment 6 | 8507 |
| Experiment 7 | 4570 |
| Experiment 8 | 8217 |

Conclusions: The introduction of peptone or excessive urea in the formula may cause poor fermentation potency. It is basically confirmed that the selections of carbon sources, nitrogen sources and comprehensive carbon and nitrogen sources in the fermenter and the feed of the present invention can be glucose, glycerol, dextrin, wheat bran, bean cake powder, raw bean powder, corn steep liquor, yeast powder.

### Example 6. Investigation of feed formula

The materials in the formula of the basic fermentation medium are glucose, bean cake powder, wheat bran, urea, ammonium sulfate, potassium dihydrogen phosphate, magnesium sulfate, potassium chloride, zinc sulfate, glycerol and polyether GPE defoamer. The formula of the feed is determined by screening based on the basic fermentation medium.

Magnesium element is an activator of many enzymes. It promotes the metabolism of carbohydrates, synthesis of nucleic acid and conversion of phosphate, etc., and thus is a base element. Because the proportion of magnesium sulfate in the basic medium is only 0.06% and only supports basic growth needs, the proportion of magnesium sulfate in the feed formula was kept unchanged at 0.06%.

The polyether GPE defoamer is only used for defoaming and does not affect the process. Thus the proportion of the polyether GPE defoamer in the formula of the feed was the same as that in the basic fermentation medium.

The bean cake powder and the wheat bran are cheap organic nitrogen sources, providing a large amount of inorganic salts and growth factors in addition to the nitrogen source. Considering the anabolism capacity of microorganisms, the optimum ratio of bean cake powder and wheat bran in the feed formula needs to be investigated.

Glycerol, as a polar protic solvent, is completely soluble in water and short-chain alcohols but insoluble in hydrocarbons. It promotes the dissolution of various non-organic acids, bases, salts and hydrophilic organic compounds in an aqueous phase. In addition, glycerol has good biocompatibility with enzymes, and can promote the correct folding of the protein, stabilize the protein structure, keep the activity of the enzymes and have a certain protective effect on cells and enzymes in the environment of heat and organic solvent. Therefore it is necessary to determine the optimum proportion of glycerol in the feed formula.

The existence of an appropriate amount of zinc sulfate has a certain promoting effect on the improvement of the fermentation titer, but excessive addition of zinc sulfate does not promote the improvement of the fermentation titer. Therefore the optimum proportion of the zinc sulfate in the formula of the feed needs investigation.

### Procedures:

The influence of bean cake powder, wheat bran, potassium dihydrogen phosphate, zinc sulfate and glycerol on the fermentation potency of mupirocin was determined according to different proportions in the experiment, and the formula with high potency would be selected as the optimum formula for the mixed feed in the feed tank during the fermentation process. The seeds were incubated in the seed tank at 20-30 °C for 24 h and then transferred to the existing fermenter for 6 days of incubation. The fermentation potency was measured.

| Factors | Zinc sulfate % | Bean cake powder % | Potassium dihydrogen phosphate % | Glycerol % | Wheat bran % |
|---|---|---|---|---|---|
| Experiment 1 | 0.05 | 1.0 | 0.05 | 0.5 | 0.5 |
| Experiment 2 | 0.05 | 2.0 | 0.075 | 1 | 1.0 |
| Experiment 3 | 0.05 | 2.5 | 0.1 | 1.5 | 1.5 |
| Experiment 4 | 0.05 | 3.0 | 0.125 | 2 | 2.0 |
| Experiment 5 | 0.125 | 1.0 | 0.075 | 1.5 | 2.0 |
| Experiment 6 | 0.125 | 2.0 | 0.05 | 2 | 1.5 |
| Experiment 7 | 0.125 | 2.5 | 0.125 | 0.5 | 1.0 |
| Experiment 8 | 0.125 | 3.0 | 0.1 | 1 | 0.5 |
| Experiment 9 | 0.2 | 1.0 | 0.1 | 2 | 1.0 |
| Experiment 10 | 0.2 | 2.0 | 0.125 | 1.5 | 0.5 |
| Experiment 11 | 0.2 | 2.5 | 0.05 | 1 | 2.0 |
| Experiment 12 | 0.2 | 3.0 | 0.075 | 0.5 | 1.5 |
| Experiment 13 | 0.275 | 1.0 | 0.125 | 1 | 1.5 |
| Experiment 14 | 0.275 | 2.0 | 0.1 | 0.5 | 2.0 |
| Experiment 15 | 0.275 | 2.5 | 0.075 | 2 | 0.5 |
| Experiment 16 | 0.275 | 3.0 | 0.05 | 1.5 | 1.0 |

16 experiments were conducted according to the above formulas, and the harvest potencies of the 16 experiments were obtained. The data was analyzed by orthogonal analysis software to obtain the influence of the five single-factor variables.

### Results (fermentation potency):

| Factors | Harvest potency (µg/mL) |
|---|---|
| Experiment 1 | 9140 |
| Experiment 2 | 9936 |
| Experiment 3 | 8783 |
| Experiment 4 | 8088 |
| Experiment 5 | 7835 |
| Experiment 6 | 10244 |
| Experiment 7 | 8832 |
| Experiment 8 | 3815 |
| Experiment 9 | 9938 |
| Experiment 10 | 7390 |
| Experiment 11 | 8073 |
| Experiment 12 | 7447 |
| Experiment 13 | 2095 |
| Experiment 14 | 8633 |
| Experiment 15 | 5835 |
| Experiment 16 | 3746 |

### Analysis:

| Factors | Zinc sulfate % | Bean cake powder % | Potassium dihydrogen phosphate % | Glycerol % | Wheat bran % |
|---|---|---|---|---|---|
| Mean 1 | 0.05 | 1.0 | 0.05 | 0.5 | 0.5 |
| Mean 2 | 0.125 | 2.0 | 0.075 | 1 | 1.0 |
| Mean 3 | 0.2 | 2.5 | 0.1 | 1.5 | 1.5 |
| Mean 4 | 0.275 | 3.0 | 0.125 | 2 | 2.0 |
| Factors | Corresponding potency (µg/mL) | | | | |
| Mean 1 | 8986.75 | 7252.00 | 7800.75 | 8513.00 | 6545.00 |
| Mean 2 | 7681.50 | 9050.75 | 7763.25 | 5979.75 | 8113.00 |
| Mean 3 | 8212.00 | 7880.75 | 7792.25 | 6938.50 | 7142.25 |
| Mean 4 | 5077.25 | 5774.00 | 6601.25 | 8526.25 | 8157.25 |

| | | | | | |
|---|---|---|---|---|---|
| Zinc sulfate: The fermentation harvest potency corresponding to mean 1 was 8986.75, which was significantly higher than those of the other three groups; thus the proportion of zinc sulfate was selected as level 1, i.e., 0.05%; Bean cake powder: The fermentation harvest potency corresponding to mean 2 was 9050.75, which was significantly higher than those of the other three groups; thus the proportion of bean cake powder was selected as level 2, i.e., 2.0%; Potassium dihydrogen phosphate: The fermentation harvest potencies corresponding to mean 1, mean 2, mean 3 and mean 4 were 7800.75, 7763.25, 7792.25 and 6601.25, respectively. The first three values had no significant difference, while mean 4 showed a significant descending trend. The harvest potency showed a descending trend along with the increase of the proportion of potassium dihydrogen phosphate, indicating that the risk of descending of the harvest potency increases when the proportion of potassium dihydrogen phosphate is higher. Thus level 1 with the minimum risk and the highest harvest potency was selected, i.e., 0.05%; Glycerol: The fermentation harvest potency corresponding to mean 1 was 8513.00, and the fermentation harvest potency corresponding to mean 4 was 8526.25. The fermentation harvest potencies corresponding to mean 1 and mean 4 were higher and had no significant difference. In view of the fact that glucose had already been used in the feed formula, glycerol and glucose were both used as carbon sources in the feed, and glucose had a greater proportion and served as the main carbon source. When glycerol was used in a proportion of 0.5% or 2%, the fermentation harvest potency had no significant difference, so the smaller proportion was selected; in addition, considering the cost-efficiency, the cost may be lower by selecting a smaller proportion, so the proportion of 0.5% was selected. Therefore the proportion of glycerol was determined as 0.5%; Wheat bran: The fermentation harvest potencies corresponding to mean 1, mean 2, mean 3 and mean 4 were 6545.00, 8113.00, 7142.25 and 8157.25, respectively. The harvest potency showed an increasing trend along with the increase of the proportion of wheat bran, indicating that the risk of descending of the harvest potency increases when the proportion of wheat bran is lower. Thus level 4 with the minimum risk and the highest harvest potency was selected, i.e., 2.0%. Conclusion: the feed formula of the present invention was determined as: 30-50% of glucose; 2.0% of wheat bran; 2.0% of bean cake powder; 0.5% of glycerol; 0.06% of magnesium sulfate; 0.05% of potassium dihydrogen phosphate; 0.05% of zinc sulfate; 0.3% of polyether GPE defoamer. | | | | | |

### Example 7. Investigation of stability of fermentation process

A scale-up verification of the fermentation process was conducted using a 4-ton fermenter in the example. According to the potency measurement results, the stability of the fermentation harvest level of the fermentation process was confirmed.

### Procedures:

The formula of the seed medium: 1.0% of glucose, 0.05% of magnesium sulfate, 0.05% of potassium dihydrogen phosphate, 0.2% of ammonium sulfate, 0.05% of polyether GPE defoamer, 0.5% of glycerol and 0.5% of wheat bran, pH 7.0.

The formula of the fermentation medium: 5.5% of glucose, 2.0% of bean cake powder, 0.5% of ammonium sulfate, 0.10% of potassium dihydrogen phosphate, 0.06% of magnesium sulfate, 0.12% of potassium chloride, 0.2% of zinc sulfate, 1.5% of glycerol, 0.3% of polyether GPE defoamer, 0.15% of urea and 1.0% of wheat bran, pH 7.0.

The formula of the feed: 30-50% of glucose, 2.0% of bean cake powder, 0.05% of potassium dihydrogen phosphate, 0.06% of magnesium sulfate, 0.05% of zinc sulfate, 0.5% of glycerol, 0.3% of polyether GPE defoamer and 2.0% of wheat bran.

Preparation of shake flask seed and preparation and sterilization of seed tank: The materials were prepared according to the above formula at a feeding volume of 220 L, and the pH was adjusted to 7.0 with sodium hydroxide solution. The tank was sterilized at 120 °C by keeping the temperature and the pressure of the tank constant for 30 min.

Samples of the seed tank medium were taken and detected before inoculation. The strain was inoculated into the seed tank inoculation and the fermenter was prepared and sterilized. The materials were prepared according to the above formula at a feeding volume of 800 L, and the pH was adjusted to 7.0 with solid sodium hydroxide. Samples of the fermenter medium were taken and detected before inoculation. The seed tank culture was conducted.

The seed in the seed tank was transferred. The saccharification solution was prepared, sterilized and detected. The fermentation culture was conducted.

Fermentation culture: The fermentation broth was incubated in the fermenter at 20-30 °C. Samples were taken every 2 h from 8 a.m. on the next day of transferring the seed to determine glucose and pH. Sugar feeding was started when glucose content was lower than 1.0%. The pH was controlled at 5.6-6.5 after sugar was fed, and glucose was controlled below 1.5%. Afterwards, regular glucose, pH and potency testing were started at 8 a.m. every day.

The pH and glucose were measured at 8 a.m. on the day of harvest and at the beginning of harvest, and the potency was determined every 2 h from 8 a.m. The fermentation culture persisted for 80-132 h.

Study design: The above scale-up experiment was repeated thrice, coded F190304FJ, F190401FJ and F1900402FJ. Growth curve was recorded using fermentation time as the horizontal axis and potency as the coordinate. The record of the growth curve started at 8 a.m. on the third day of transferring the seed. The potency was determined every 4 h. The pH and glucose were measured at 8 a.m. and 4 p.m. every day. The pH and glucose were measured at 8 a.m. on the day of harvest and at the beginning of harvest, and the potency was determined every 2 h from 12 p.m. until harvest. Conclusion: As can be seen in FIG. 1, the harvest potency of the three scale-up experiments stably reached 8000 µg/mL, suggesting that the production process is stable and controllable.

## Claims

1. A fermentation method for mupirocin, comprising: fermentation culture and feed culture, wherein a medium added in the feed culture comprises, in mass-to-volume ratio, 25-60% of a carbon source, 1-10% of a nitrogen source, 0.10-0.25% of an inorganic salt, 0.1-0.5% of a defoamer, the carbon source is selected from one or more of glucose, sucrose, fructose, glycerol, dextrin, peptone, wheat bran, bean cake powder, corn starch, soybean powder, potato powder, tapioca starch, corn steep liquor, yeast powder or mannitol; the nitrogen source is selected from one or more of ammonia solution, urea, ammonium salt, nitrate, peptone, wheat bran, corn steep liquor, bean cake powder, peanut cake powder, cottonseed cake powder or yeast powder; the inorganic salt is selected from sulfate and/or phosphate.

2. The fermentation method for mupirocin according to claim 1, wherein the carbon source is selected from one or more of glucose, glycerol, dextrin, wheat bran, bean cake powder, corn steep liquor or yeast powder; the nitrogen source is selected from one or more of wheat bran, bean cake powder, corn steep liquor or yeast powder.

3. The fermentation method for mupirocin according to claim 2, wherein the medium added in the feed culture comprises, in mass-to-volume ratio, 25-60% of glucose, 1-5% of bean cake powder, 1-5% of wheat bran, 0.1-1.0% of glycerol;
or, 25-60% of glucose, 1-5% of yeast powder, 1-5% of wheat bran, 0.1-1.0% of glycerol;
or, 25-60% of glucose, 1-5% of raw bean powder, 1-5% of wheat bran, 0.1-1.0% of glycerol;
or, 25-60% of glucose, 1-5% of bean cake powder, 1-5% of dextrin, 0.1-1.0% of glycerol;
or, 25-60% of glucose, 1-5% of bean cake powder, 1-5% of corn steep liquor, 0.1-1.0% of glycerol;
or, 25-60% of glucose, 1-5% of raw bean powder, 1-5% of dextrin, 0.1-1.0% of glycerol.

4. The fermentation method for mupirocin according to claim 1, wherein the inorganic salt in the medium added in the feed culture is potassium dihydrogen phosphate, magnesium sulfate and zinc sulfate; the medium added in the feed culture comprises, in mass-to-volume ratio, 0.01-0.1% of potassium dihydrogen phosphate, 0.01-0.1% of magnesium sulfate, 0.01-0.1% of zinc sulfate.

5. The fermentation method for mupirocin according to claim 1, wherein the defoamer in the medium added in the feed culture is polyether GPE defoamer; the medium added in the feed culture comprises, in mass-to-volume ratio, 0.1-0.5% of polyether GPE defoamer.

6. The fermentation method for mupirocin according to claim 1, wherein the pH in the feed culture is 5.0-6.5.

7. The fermentation method for mupirocin according to claim 1, wherein a medium added in the fermentation culture comprises, by mass percentage: 5.0-12.0% of a carbon source, 2.0-10.0% of a nitrogen source, 0.10-1.5% of an inorganic salt, 0.1-0.5% of a defoamer;
the carbon source and the nitrogen source are selected from one or more of glucose, glycerol, dextrin, wheat bran, bean cake powder, raw bean powder, corn steep liquor, yeast powder;
and/or, the inorganic salt is selected from sulfate, phosphate, and chloride;
and/or, the pH in the fermentation culture is 6.0-7.5, preferably 6.8-7.5;
and/or, the feeding volume in the fermentation culture may be 0-20000 L;
and/or, the time of the fermentation tank culture is 48-144 h, preferably 80-132 h;
and/or, the temperature of the fermentation tank is 10-37 °C, preferably 20-30 °C.

8. The fermentation method for mupirocin according to claim 1, specifically comprising:
a. shake flask seed culture: preparing a shake flask seed at pH 6.0-7.5, split charging, sterilizing, inoculating the shake flask seed into a seed shake flask at an inoculation amount of 0.1-10.0%, incubating at 20-30 °C for 12-48 h to obtain a shake flask seed bacterial solution;
b. seed tank culture: adjusting the seed obtained in step a to pH 6.0-7.5, sterilizing, incubating the bacterial solution for 12-36 h at a tank temperature of 20-30 °C to obtain a seed bacterial solution;
c. fermentation culture: inoculating the seed bacterial solution obtained in step b into a fermenter for fermentation culture at a temperature of 20-30 °C, adjusting the pH to 6.8-7.5 monitoring the pH and the content of glucose and feeding, collecting the product to obtain a mupirocin fermentation broth.

9. The fermentation method for mupirocin according to claim 8, wherein the seed bacterial solution inoculated in the fermentation culture is obtained by 1-2 levels of seed cultures, the inoculation amount of the seed bacterial solution is 1.0-8.0% by volume.

10. The fermentation method for mupirocin according to any one of claims 7-9, wherein the medium in the fermentation culture comprises, in mass-to-volume ratio: 5.0-6.0% of glucose, 1.5-3.0% of bean cake powder, 0.5-2.5% of wheat bran, 0.1-0.2% of urea, 1.0-2.0% of glycerol, 0.01-1.0% of ammonium sulfate, 0.01-0.25% of potassium dihydrogen phosphate, 0.01-0.25% of magnesium sulfate, 0.01-0.25% of potassium chloride, 0.01-0.25% of zinc sulfate, 0.1-0.5% of polyether GPE defoamer;
Or, 5.0-6.0% of glucose, 1.5-3.0% of yeast powder, 0.5-2.5% of wheat bran, 0.1-0.2% of urea, 1.0-2.0% of glycerol, 0.01-1.0% of ammonium sulfate, 0.01-0.25% of potassium dihydrogen phosphate, 0.01-0.25% of magnesium sulfate, 0.01-0.25% of potassium chloride, 0.01-0.25% of zinc sulfate, 0.1-0.5% of polyether GPE defoamer;
Or, 5.0-6.0% of glucose, 1.5-3.0% of raw bean powder, 0.5-2.5% of wheat bran, 0.1-0.2% of urea, 1.0-2.0% of glycerol, 0.01-1.0% of ammonium sulfate, 0.01-0.25% of potassium dihydrogen phosphate, 0.01-0.25% of magnesium sulfate, 0.01-0.25% of potassium chloride, 0.01-0.25% of zinc sulfate, 0.1-0.5% of polyether GPE defoamer;
Or, 5.0-6.0% of glucose, 1.5-3.0% of bean cake powder, 0.5-2.5% of dextrin, 0.1-0.2% of urea, 1.0-2.0% of glycerol, 0.01-1.0% of ammonium sulfate, 0.01-0.25% of potassium dihydrogen phosphate, 0.01-0.25% of magnesium sulfate, 0.01-0.25% of potassium chloride, 0.01-0.25% of zinc sulfate, 0.1-0.5% of polyether GPE defoamer;
Or, 5.0-6.0% of glucose, 1.5-3.0% of bean cake powder, 0.5-2.5% of corn steep liquor, 0.1-0.2% of urea, 1.0-2.0% of glycerol, 0.01-1.0% of ammonium sulfate, 0.01-0.25% of potassium dihydrogen phosphate, 0.01-0.25% of magnesium sulfate, 0.01-0.25% of potassium chloride, 0.01-0.25% of zinc sulfate, 0.1-0.5% of polyether GPE defoamer;
Or, 5.0-6.0% of glucose, 1.5-3.0% of raw bean powder, 0.5-2.5% of dextrin, 0.1-0.2% of urea, 1.0-2.0% of glycerol, 0.01-1.0% of ammonium sulfate, 0.01-0.25% of potassium dihydrogen phosphate, 0.01-0.25% of magnesium sulfate, 0.01-0.25% of potassium chloride, 0.01-0.25% of zinc sulfate, 0.1-0.5% of polyether GPE defoamer.

11. A medium for use in preparing mupirocin, wherein the medium comprises, in mass-to-volume ratio: 25-60% of glucose, 1-5% of bean cake powder, 1-5% of wheat bran, 0.1-1.0% of glycerol, 0.01-0.1% of potassium dihydrogen phosphate, 0.01-0.1% of magnesium sulfate, 0.01-0.1% of zinc sulfate, 0.1-0.5% of polyether GPE defoamer;
Or, wherein the medium of the feed culture comprises 25-60% of glucose, 1-5% of yeast powder, 1-5% of wheat bran, 0.1-1.0% of glycerol, 0.01-0.1% of potassium dihydrogen phosphate, 0.01-0.1% of magnesium sulfate, 0.01-0.1% of zinc sulfate, 0.1-0.5% of polyether GPE defoamer;
Or, wherein the medium of the feed culture comprises 25-60% of glucose, 1-5% of raw bean powder, 1-5% of wheat bran, 0.1-1.0% of glycerol, 0.01-0.1% of potassium dihydrogen phosphate, 0.01-0.1% of magnesium sulfate, 0.01-0.1% of zinc sulfate, 0.1-0.5% of polyether GPE defoamer;
Or, wherein the medium of the feed culture comprises 25-60% of glucose, 1-5% of bean cake powder, 1-5% of dextrin, 0.1-1.0% of glycerol, 0.01-0.1% of potassium dihydrogen phosphate, 0.01-0.1% of magnesium sulfate, 0.01-0.1% of zinc sulfate, 0.1-0.5% of polyether GPE defoamer;
Or, wherein the medium of the feed culture comprises 25-60% of glucose, 1-5% of bean cake powder, 1-5% of corn steep liquor, 0.1-1.0% of glycerol, 0.01-0.1% of potassium dihydrogen phosphate, 0.01-0.1% of magnesium sulfate, 0.01-0.1% of zinc sulfate, 0.1-0.5% of polyether GPE defoamer;
Or, wherein the medium of the feed culture comprises 25-60% of glucose, 1-5% of raw bean powder, 1-5% of dextrin, 0.1-1.0% of glycerol, 0.01-0.1% of potassium dihydrogen phosphate, 0.01-0.1% of magnesium sulfate, 0.01-0.1% of zinc sulfate, 0.1-0.5% of polyether GPE defoamer.
